# EUROPEAN PATENT SPECIFICATION

(11) **EP 0 479 117 B1**
(45) Date of publication and mention of the grant of the patent: **28.11.2001**
(21) Application number: 91116396.2
(22) Date of filing: 26.09.1991
(51) Int. Cl.: C12Q 1/68, C07H 21/00

(54) **Methods and reagents for identifying gram negative bacteria**
Verfahren und Reagentien für die Identifikation von Bakterien
Méthodes et réactifs pour l'identification bactérienne

(30) Priority: 05.10.1990 US 593176; 06.05.1991 US 696448
(43) Date of publication of application: 08.04.1992
(73) Proprietor: F. HOFFMANN-LA ROCHE AG, 4002 Basel (CH)
(72) Inventor: Leong, Diane Uratsu, Berkeley, CA 94707 (US)
(74) Representative: Knauer, Martin, Dr.

(56) References cited:
- EP-A- 0 272 009
- WO-A-90/01560
- WO-A-90/11370
- WO-A-90/15157
- WO-A-92/06202
- US-A- 4 977 251
- JOURNAL OF BACTERIOLOGY vol. 164, no. 1, October 1985, BALTIMORE US pages 230 - 236; W. G. WEISBURG ET AL: 'Natural relationship between Bacteroides and Flavobacteria'
- FEBS LETTERS. vol. 94, no. 1, October 1978, AMSTERDAM NL pages 152 - 156; P. CARBON ET AL: 'The sequence of E. coli ribosomal 16 S RNA determined by new rapid gel methods'
- JOURNAL OF CLINICAL MICROBIOLOGY vol. 28, no. 9, September 1990, WASHINGTON US pages 1942 - 1946; H. W. KENNETH ET AL: 'Amplification of bacterial 16S ribosomal DNA with polymerase chain reaction'
- GEMS MICROBIOLOGY LETTERS VOL 57, 1989 P.19-24

## Description

The present invention relates generally to methods and reagents for identifying and detecting gram-negative bacteria causing septicemia.

In order to successfully treat a disease caused by a bacterium the rapid and accurate detection and identification of the disease-causing bacterium is required. The detection and identification has traditionally been accomplished by pure culture isolation, followed by identification procedures that make use of knowledge of specimen source, growth requirements, visible (colony) growth features, microscopic morphology, staining reactions, and biochemical characteristics.

An important step in determining the identity of a bacterium is the Gram stain. This procedure involves treating a heat-fixed bacterial smear on a glass slide with the basic dye, crystal violet. All organisms take up the dye. The smear is then covered with Gram's iodine solution (3 percent iodine-potassium iodide in water or a weak buffer, pH 8.0, in order to neutralize acidity formed from iodine on standing). After a water rinse and decolorization with acetone, the preparation is washed thoroughly in water and counterstained with a red dye, usually safranin. The stained preparation is then rinsed with water, dried, and examined under oil using a light microscope.

Most bacteria can be differentiated into two groups by this stain. Gram-positive organisms stain blue, whereas about one-third of the cocci, one-half of the bacilli, and all spiral organisms stain red and are said to be gram-negative. This method, while effective, is very time consuming and involves many different procedures which present many opportunities for error. The Gram stain and other culture-based methods of detection require incubation of the sample with culture medium at least overnight in order to obtain a pure culture.

The presence of bacteria or fungi in the blood, commonly referred to as septicemia, can have severe and life-threatening clinical consequences. Septicemia can result in septic shock, which includes the following symptoms - hypotension, lactic acidosis, hypoxemia, oligouria, confusion, disseminated intravascular coagulation, gastrointestinal bleeding, disturbances of metabolism, and subtle skin lesions. As little as one colony-forming unit (CFU) may be present in a 30 ml blood sample in a patient with septicemia. Since culture is currently the most sensitive and commonly used method of detecting bacteria or fungi in the blood, treatment of suspected septicemia is often begun empirically, without waiting for the results of culture. It is clear that a rapid diagnostic method for detecting bacteria in the blood with the same sensitivity as culture would be a significant improvement over currently used methods.

Therefore, the present invention provides methods and reagents for the rapid detection and identification of bacteria causing septicemia. The detection is based upon the hybridization of nucleotide probes to nucleotide sequences as well as transcripts therefrom present in defined species or group of species but not in others.

In a preferred embodiment, a target region from genomic DNA or from a reverse transcript of 16S rRNA is amplified and the resultant amplified DNA is treated with a panel of probes which can hybridize to the DNA of a species or group of species of bacteria but not to others. The probes which sucessfully hybridize to the amplified DNA are determined and the bacterium is classified as gram-negative or as a particular species or group of species depending on which probes hybridize to the amplified DNA.

Also defined and claimed herein are specific probes and their complements for identifying gram-negative bacteria causing septicemia.

The invention further contemplates the formulation and use of Polymerase Chain Reaction (PCR) kits containing universal bacterial primers for amplifying a specific universal target region of DNA for all bacteria and a panel of probes which hybridize to a nucleotide sequence which is unique to a species or group of species of bacteria within that target region.

### Brief Description of the Figures

Fig. 1 shows two universal bacterial primers DG74 and RW01 which can be used to amplify a target region by PCR in gram-positive, gram-negative and other bacteria.

Fig. 2 shows a gram-positive specific probe RW03.

Fig. 3 shows four candidate gram-negative probes, RW04, DL04, DL05, and RDR278.

Fig. 4 shows two candidate universal bacterial probes RDR244-and RDR245.

Fig. 5 shows a Escherichia coli/enteric bacteria probe.

Fig. 6 shows a Bacteroides probe.

Table 1 summarizes the hybridization data on the Gram-negative probes RW04 and DL04.

Table 2 summarizes the results of testing probes RDR278 and DL04, the Gram-negative probes; RDR244 and RDR245, two candidate universal bacterial probes; and RDR279, the Bacteroides probe.

Table 3 shows a list of organisms tested with RDR244 and RDR245.

Table 4 shows a summary of data obtained with the E.coli/enteric bacteria probe RDR140KG.

### Detailed Description of the Invention

The present invention relates to a method for determining the presence of and identification of bacteria by means of hybridizing probes to nucleotide sequences which are unique to gram-negative bacteria or to a species or group of species of gram-negative bacteria.

The use of specific polynucleotide sequences as probes for the recognition of infectious agents is becoming a valuable alternative to problematic immunological identification assays. For example, PCT publication W084/02721, published 19 July 1984 describes the use of nucleic acid probes complementary to targeted nucleic acid sequences composed of ribosomal RNA, transfer RNA, or other RNA in hybridization procedures to detect the target nucleic acid sequence. While the assay may provide greater sensitivity and specificity than known DNA hybridization assays, hybridization procedures which require the use of a complementary probe are generally dependent upon the cultivation of a test organism and are, therefore, unsuitable for rapid diagnosis. Probes can be used directly on clinical specimens if a means of amplifying the DNA target is available.

PCT Patent Publication No. 90/15157 discloses a method of detecting the presence of eubacteria using as primers and/or probes a number of 16S rRNA gene derived sequences, which are broadly specific for all eubacteria or for certain specific subgroups of eubacteria, e.g. gram-positive or gram-negative bacteria. One such probe, probe 1746, corresponds to positions 1188 to 1216 of the E. coli 16S rRNA and hybridizes preferentially to gram-positive bacteria (cf. page 21, lines 8-9 and page 27).

PCT Patent Publication No. 90/01560 discloses nucleic acid probes which hybridize with RNA of several major classes of bacteria but not with that of mammalian cell.

For use in the present invention, probes for bacterial species or groups of species causing septicemia include :
Gram-negative probes
Bacteroides probes

These probes would be useful in hybridizing to DNA or RNA amplified by the Polymerase Chain Reaction (PCR). PCR is a powerful technique that can be used for the detection of small numbers of pathogens, whose in vitro cultivation is difficult or lengthy, or as a substitute for other methods which require the presence of living specimens for detection. In its simplest form, PCR is an in vitro method for the enzymatic synthesis of specific DNA sequences, using two oligonucleotide primers that hybridize to opposite strands and flank the region of interest in the target DNA. A repetitive series of cycles involving template denaturation, primer annealing, and the extension of the annealed primers by DNA polymerase results in the exponential accumulation of a specific fragment whose termini are defined by the 5' ends of the primers. PCR reportedly is capable of producing a selective enrichment of a specific DNA sequence by a factor of 10¹². The PCR method is described in Saiki et al., (1985) Science 230, 1350-1354 and is the subject of U.S. Patent Nos. 4,683,195, 4,683,202 and 4,800,159. This method has been used to detect the presence of the aberrant sequence in the beta-globin gene which is related to sickle cell anemia (Saiki et al., (1985) supra) and the human immunodeficiency virus (HIV) RNA (Byrne et al., (1988) Nuc. Acids Res. 16, 4165). However, before the method can be used, enough of the nucleotide sequence of the disease-associated polynucleotide must be known to design primers for the amplification, and to design probes specific enough to detect the amplified product.

The invention provides a method for determining the presence of a bacterial polynucleotide in samples suspected of containing said polynucleotide, wherein said polynucleotide contains a selected target region, said method comprising:
(a) amplifying the target region, if any, to a detectable level;
(b) incubating the amplified target region, if any, with a probe under conditions which allow specificity of hybrid duplexes; and
(c) detecting hybrids formed between the amplified target region, if any, and the probe.

The method of the present invention thus enables determination of the presence of bacteria more rapidly than heretofore possible with prior art detection methods. The basic PCR process is carried out as follows.

A sample is provided which is suspected of containing a particular nucleic acid sequence of interest, the "target sequence". The nucleic acid contained in the sample may be first reverse transcribed into cDNA using Tth DNA polymerase as purified enzyme, if necessary, and then denatured, using any suitable denaturing method, including physical, chemical, or enzymatic means, which are known to those of skill in the art. A preferred physical means for strand separation involves heating the nucleic acid until it is completely (>99%) denatured. Typical heat denaturation involves temperatures ranging from about 80°C to about 150°C, for times ranging from about 5 seconds to 10 minutes using current technology.

The denatured DNA strands are then incubated with the selected oligonucleotide primers under hybridization conditions, conditions which enable the binding of the primers to the single oligonucleotide strands. As known in the art, the primers are selected so that their relative positions along a duplex sequence are such that an extension product synthesized from one primer, when it is separated from its complement serves as a template for the extension of the other primer to yield a replicate chain of defined length.

The primer must be sufficiently long to prime the synthesis of extension products in the presence of the agent for polymerization. The exact length of the primers will depend on many factors, including temperature, source of the primer and use of the method. For example, depending on the complexity of the target sequence, the oligonucleotide primer typically contains about 15-30 nucleotides, although it may contain more or fewer nucleotides. Short primer molecules generally require cooler temperatures to form sufficiently stable hybrid complexes with the template. The primers must be sufficiently complementary to selectively hybridize with their respective strands.

The primers used herein are selected to be "substantially" complementary to the different strands of each specific sequence to be amplified. The primers need not reflect the exact sequence of the template, but must be sufficiently complementary to selectively hybridize with their respective strands. Non-complementary bases or longer sequences can be interspersed into the primer, or the primer can contain a subset complementary to the specific sequence provided that the primer retains sufficient complementarity with the sequence of one of the strands to be amplified to hybridize therewith, and to thereby form a duplex structure which can be extended by the polymerizing means. The non-complementary nucleotide sequences of the primers may include restriction enzyme sites. Appending a restriction enzyme site to the end(s) of the target sequence is particularly helpful for subsequent cloning of the target sequence.

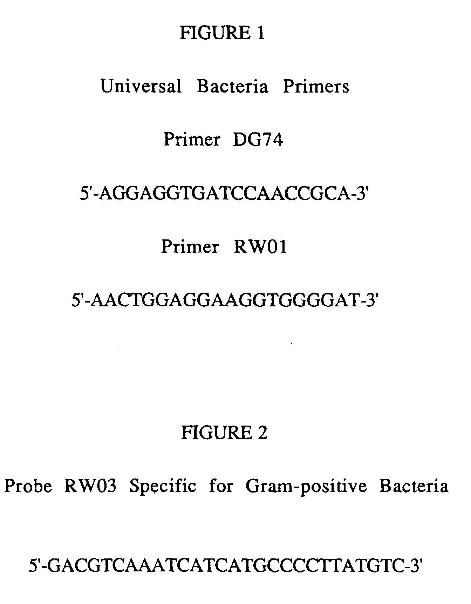

The oligonucleotide primers and probes for use in the present invention are shown in Figures 1-6. They may be prepared by any suitable method. Methods for preparing oligonucleotides of specific sequence are known in the art, and include, for example, cloning and restriction of appropriate sequences, and direct chemical synthesis. The primers may be labeled, if desired, by incorporating means detectable by spectroscopic, photochemical, biochemical, immunochemical, or chemical means.

Template-dependent extension of the oligonucleotide primer(s) is then catalyzed by a polymerizing agent in the presence of adequate amounts of the four deoxyribonucleoside triphosphates (dATP, dGTP, dCTP, and dTTP) or analogs, in a reaction medium which is comprised of the appropriate salts, metal cations, and pH buffering system. Suitable polymerizing agents are enzymes known to catalyze primer and template-dependent DNA synthesis. Known DNA polymerases include, for example, E. coli DNA polymerase I or its Klenow fragment, T₄ DNA polymerase, Taq DNA polymerase, Tth DNA polymerase from Thermus thermophilus and DNA polymerase from Thermococcus litoralis. The reaction conditions for catalyzing DNA synthesis with these DNA polymerases are well known in the art.

The products of the synthesis are duplex molecules consisting of the template strands and the primer extension strands, which include the target sequence. These products, in turn, serve as templates for another round of replication. In the second round of replication, the primer extension strand of the first cycle is annealed with its complementary primer; synthesis yields a "short" product which is bounded on both the 5'-and the 3'-ends by primer sequences or their complements. Repeated cycles of denaturation, primer annealing, and extension result in the exponential accumulation of the target region defined by the primers. Sufficient cycles are run to achieve the desired amount of polynucleotide containing the target region of nucleic acid. The desired amount may vary, and is determined by the function which the product polynucleotide is to serve.

The PCR method can be performed in a number of temporal sequences. For example, it can be performed step-wise, where after each step new reagents are added, or in a fashion where all of the reagents are added simultaneously, or in a partial step-wise fashion, where fresh reagents are added after a given number of steps.

In a preferred method, the PCR reaction is carried out as an automated process which utilizes a thermostable enzyme. In this process the reaction mixture is cycled through a denaturing step, a primer annealing step, and a synthesis step. A DNA thermal cycler specifically adapted for use with a thermostable enzyme may be employed, which utilizes temperature cycling without a liquid-handling system, thereby eliminating the need to add the enzyme at every cycle. This type of machine is commercially available.

After amplification by PCR, the target polynucleotides may be detected directly by gel analysis provided the target DNA is efficiently amplified and the primers are highly specific to the target region to be amplified. To assure PCR efficiency, glycerol and other related solvents such as dimethyl sulfoxide, can be used to increase the sensitivity of the PCR at the amplification level and to overcome problems pertaining to the sequencing of regions of DNA having a strong secondary structure. These problems may include (1) low efficiency of the PCR, due to a high frequency of templates that are not fully extended by the polymerizing agent or (2) incomplete denaturation of the duplex DNA at high temperature, due to high GC content. The use of such solvents can increase the sensitivity of the assay at the level of amplification to approximately several femtograms of DNA (which is believed to correspond to a single bacterial cell). This level of sensitivity eliminates the need to detect amplified target DNA using a probe, and thereby dispenses with the requirements for radioactive probes, gel electrophoresis, Southern blotting, filter hybridization, washing and autoradiography. The concentration range for glycerol is about 5%-20% (v/v), and the DMSO concentration range is about 3% - 10% (v/v).

Alternatively, and in accordance with the present invention the target polynucleotides may be detected by hybridization with a probe polynucleotide which forms a stable hybrid with that of the target sequence under stringent to low stringency hybridization and wash conditions. If it is expected that the probes will be completely complementary (i.e., about 99% or greater) to the target sequence, stringent conditions will be used. If some mismatching is expected, for example if variant strains are expected with the result that the probe will not be completely complementary, the stringency of hybridization may be lessened. However, conditions are chosen which rule out nonspecific/adventitious binding. Conditions which affect hybridization and which select against nonspecific binding are known in the art. Generally, lower salt concentration and higher temperature increase the stringency of binding. For example, it is usually considered that stringent conditions are incubation in solutions which contain approximately 0.1 x SSC, 0.1% SDS, at about 65°C incubation/ wash temperature, and moderately stringent conditions are incubation in solutions which contain approximately 1-2 X SSC, 0.1% SDS and about 50°-65°C incubation/wash temperature. Low stringency conditions are 2 X SSC and about 30°-50°C.

An alternate method of hybridization and washing is to perform a low stringency hybridization (5x SSPE, 0.5% SDS) followed by a high stringency wash in the presence of 3M tetramethylammonium chloride (TMACl). The effect of the TMACl is to equalize the relative binding of A-T and G-C base pairs so that the efficiency of hybridization at a given temperature is a function of the length of the polynucleotide. Using TMACl, it is possible to vary the temperature of the wash to achieve the level of stringency desired. (See Base composition-independent hybridization in tetramethylammonium chloride: A method for oligonucleotide screening of highly complex gene libraries; Wood, et al. (1985) Proc. Natl. Acad. Sci. USA 82, 1585-1588).

Probes for bacterial target sequences may be derived from the 16S rRNA gene sequences or their complements. The probes may be of any suitable length which span the target region, but which exclude the primers, and which allow specific hybridization to the target region. Generally, the probes will have at least 14 nucleotides, preferably at least 18 nucleotides, and more preferably at least 20 to 30 nucleotides of either of the complementary DNA strands. In fact, the target sequence can come from either complementary DNA strands. If there is to be complete complementarity, i.e., if the strain contains a sequence identical to that of the probe, since the duplex will be relatively stable under even stringent conditions, the probes may be short, i.e., in the range of about 10-30 basepairs. If some degree of mismatch is expected with the probe, i.e., if it is suspected that the probe will hybridize to a variant region, the probe may be of greater length, since length seems to counterbalance some of the effect of the mismatch(es). The probe may be formed from a subset of the target region and therefore need not span the entire target region. Any subset of the target region can be used in constructing the probe provided the probe by hybridizing to that portion of the target region will specifically identify the target region. If desired, the probe may also be labeled. A variety of labels which would be appropriate, as well as methods for their inclusion in the probe are known in the art, and include, for example, radioactive atoms, such as ³²P, or other recognizable functionalities, e.g., biotin (preferably using a spacer arm), fluorescent dyes, electron-dense reagents, enzymes capable of forming easily detectable reaction products (e.g., alkaline phosphatase, and horseradish peroxidase), or antigens for which specific antisera or monoclonal antibodies are available.

Analysis of the nucleotide sequence of the target region may be by direct analysis of the PCR amplified products as described in Gyllensten and Erlich, (1988) Proc. Natl. Acad. Sci. USA 85, 7652-7656.

It may be desirable to determine the length of the PCR product detected by the probe. This may be particularly true if it is suspected that variant bacterial strains may contain deletions or insertions within the target region, or if one wishes to confirm the length of the PCR product. In such circumstances, it is preferable to subject the products to size analysis as well as hybridization with the probe. Methods for determining the size of nucleic acids are known in the art, and include, for example, gel electrophoresis, sedimentation in gradients, and gel exclusion chromatography.

The presence of the target sequence in a biological sample is detected by determining whether a hybrid has been formed between the probe and the nucleic acid subjected to the PCR amplification techniques. Methods to detect hybrids formed between a probe and a nucleic acid sequence are well-known in the art. For example, an unlabeled sample may be transferred to a solid matrix to which it binds, and the bound sample subjected to conditions which allow specific hybridization with a labeled probe; the solid matrix is then examined for the presence of the labeled probe. Alternatively, if the sample is labeled, an unlabeled probe is bound to the matrix, and after exposure to the appropriate hybridization conditions, the matrix is examined for the presence of a label. Saiki et al., (1988) Proc. Natl. Acad. Sci. USA 86, 6230-6234 describe methods of immobilizing multiple probes on a solid support and using hybridization to detect the amplified target polynucleotides of interest. The latter procedure is well suited to the use of a panel of probes which can provide different levels of identification of an amplified target DNA, depending on the type of information desired. In another alternative procedure, a solution phase sandwich assay may be used with labeled polynucleotide probes, and the methods for the preparation of such probes are described in U.S. Patent No. 4,820,630, issued April 11, 1989.

Also within the scope of the present invention are PCR kits for use in carrying out any of the aforementioned PCR processes. The PCR kits for the detection of bacteria comprise a first container and a second container wherein the first container contains primers capable of amplifying a target region of a polynucleotide sequence within bacteria, and the second container contains one or more probes capable of hybridizing to the amplified target nucleic acid sequence. Preferably, the primers used are these as shown in Figure 1 and the probe is selected from the probes shown in Figures 2-6. Either of these may or may not be labeled. If unlabeled, the ingredients for labeling may also be included in the kit. The kit may also contain other suitably packaged reagents and material needed for the particular hybridization protocol, for example, standards, and/or polymerizing agents, as well as instruction for conducting the test.

In use, the components of the PCR kit, when applied to a nucleic acid sample, create a reagent mixture which enables the detection and amplification of the target nucleic acid sequence. The reagent mixture thus includes the components of the kit as well as a nucleic acid sample which contains the polynucleotide chain of interest.

By way of further specificity, the following probe and primer nucleotide base pair data is provided:

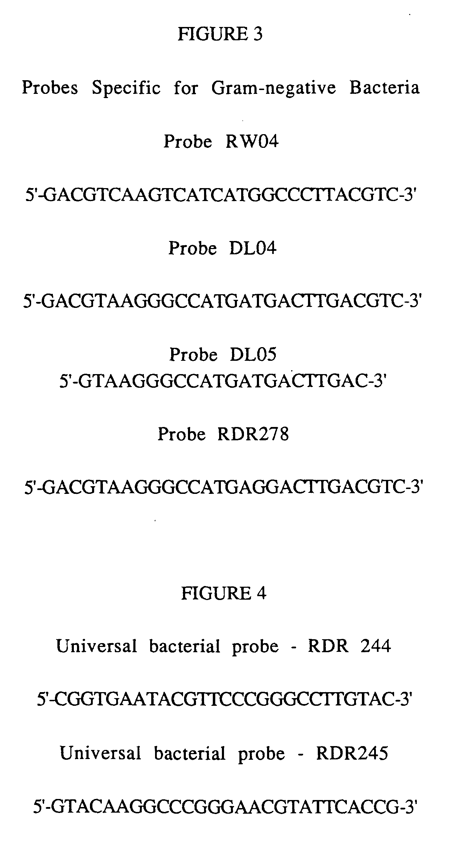

Probe RDR245, Fig. 4, corresponds to the complement of nucleotide base numbers 1369-1395 in the E. coli. 16S ribosomal RNA gene as specified in the reference of Neefs et al. (infra)

Primer RW01, Fig. 1, corresponds to nucleotide base numbers 1170-1189 in the E. coli 16S ribosomal RNA gene as specified in Neefs reference.

Primer DG74, Fig. 1, corresponding to the complement of nucleotide base numbers 1522-1540 in the E. coli 16S ribosomal RNA gene as specified in Neefs reference.

Probe RW03, Fig. 2, corresponding to nucleotide base number 1190-1217 in the E. coli 16S ribosomal RNA gene as specified in Neefs reference.

Probes DL04 and RDR278, Fig. 3, corresponding to the complement of nucleotide base number 1190-1217 in the E. coli 16S ribosomal RNA gene as specified in Neefs reference.

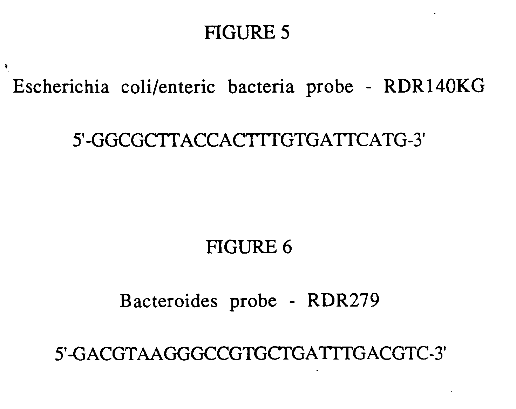

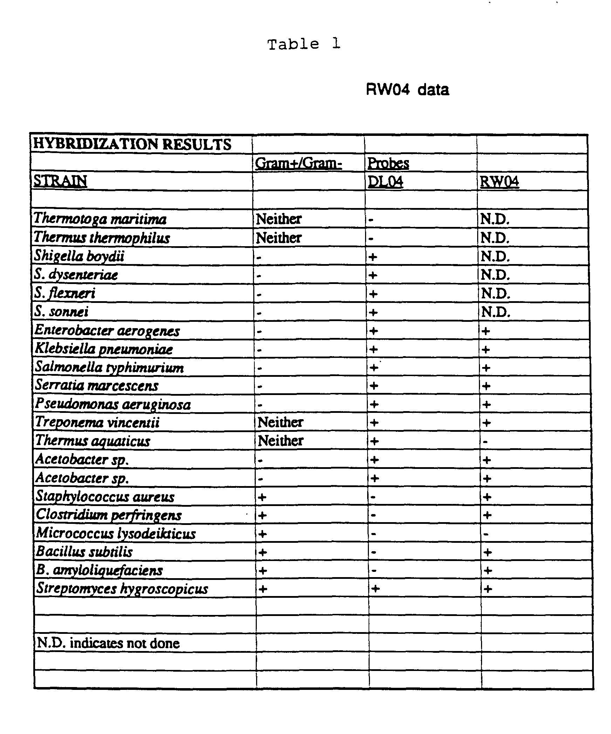

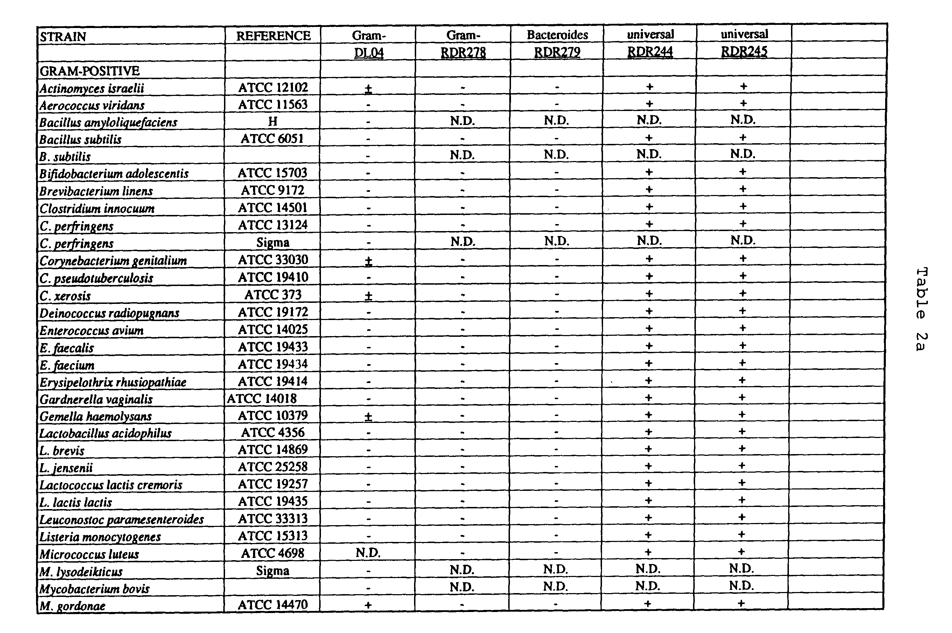

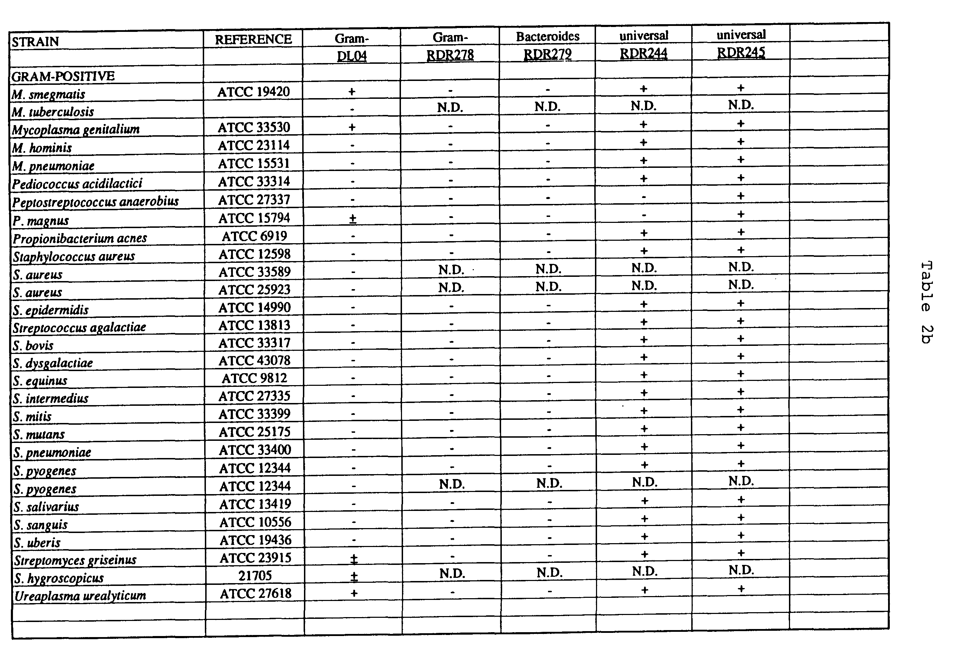

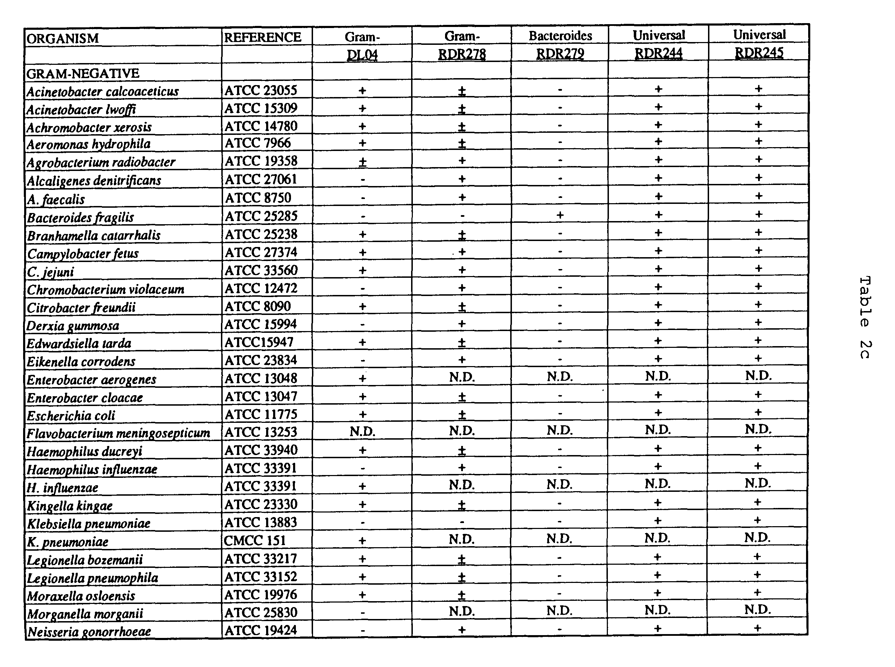

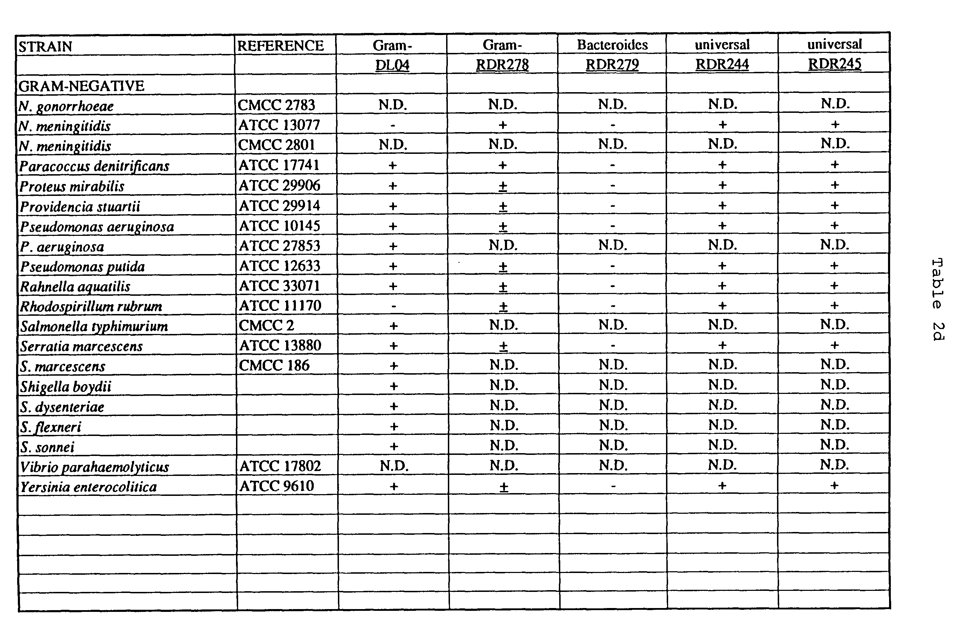

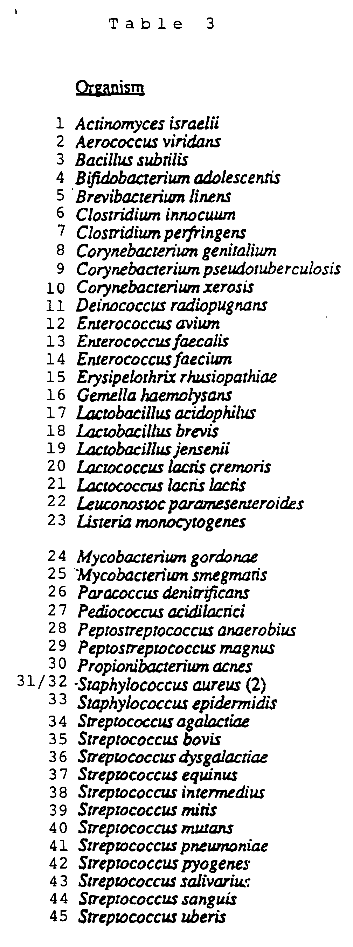

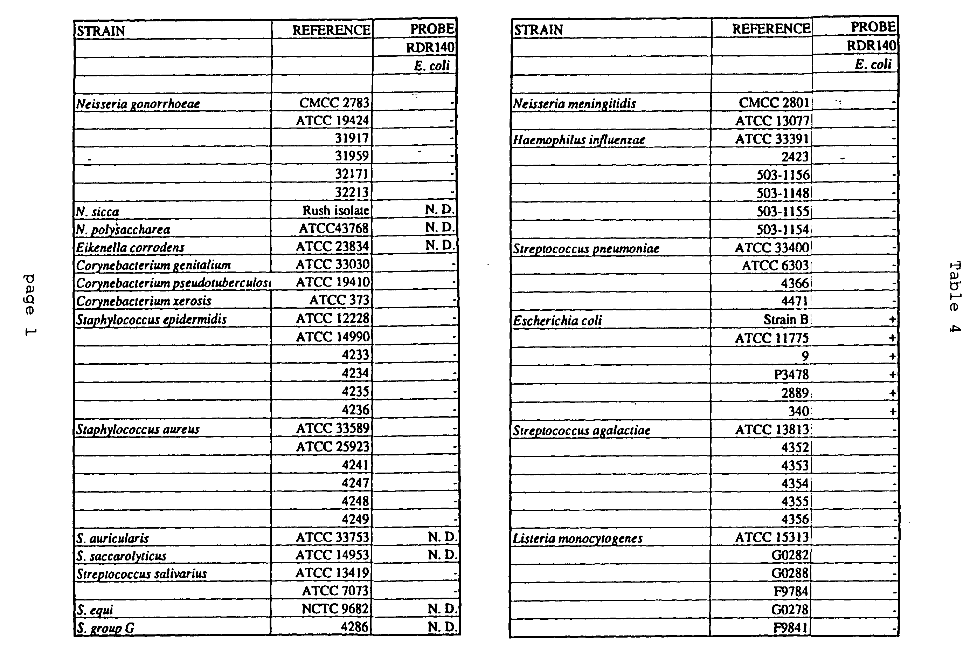

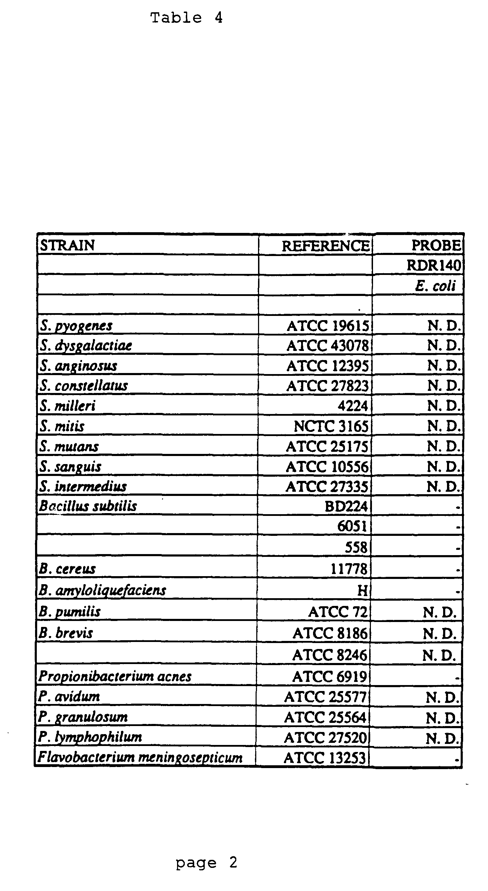

Probe RDR140 KG, Fig. 5, corresponding to nucleotide base number 1458-1482 in the E. coli 16S ribosomal RNA gene as specified in Neefs reference.

Probe RDR279, Fig. 6, corresponding to the complement of nucleotide base number 1190-1217 in the E. coli 16S ribosomal RNA gene as specified in Neefs reference.

Oligonucleotide probes based on the 16S rRNA gene for the detection of nucleic acids from various microorganisms have been described in the scientific literature. For example, universal bacterial probes have been described by Wilson, et al ("Amplification of bacterial 16S ribosomal DNA with polymerase chain reaction", Kenneth Wilson, Rhonda Blitchington, and Ronald Greene (1990), Journal of Clinical Microbiology, 28, 1942-1946) and Chen, et al ("Broad range DNA probes for detecting and amplifying eubacterial nucleic acids", Kui Chen, Harold Neimark, Peter Rumore, and Charles Steinman, (1989) FEMS microbiology letters, 57, 19-24). Examples of genus- and species-specific probes have been described by Barry, et al ("A general method to generate DNA probes for microorganisms", Tom Barry, Richard Powell, Frank Gannon (1990), Biotechnology 8, 233-236), Atlas and Bej ("Detecting bacterial pathogens in environmental water samples by using PCR and gene probes", Ronald Atlas and Asim Bej, in "PCR protocols: A guide to methods and applications," (1990) pp. 399-406 (Jnis M.A., ed.), Academic Press, Inc.), and in Genprobe international patent application with Publ. No. W088/03957. The invention claimed in this application differs from these inventions in the range of bacteria detected. The gram-positive and gram-negative probes detect a range of different bacterial genera and are therefore more specific than universal bacterial probes and more broad than genus- or species-specific probes. Using a panel including a universal bacterial probe, gram-positive and gram-negative probes and species or group specific probes, it is possible to obtain a more reliable detection of a bacterium than the use of a collection of genus- or species-specific probes, since it is possible for the universal bacterial and gram-negative or gram-positive probes to detect bacteria which are not detected by any of the more specific probes. The panel described also provides more clinically useful information than a single universal bacterial probe; since different antibiotic therapy is recommended for gram-negative versus gram-positive bacterial infections.

The following examples are intended to be illustrative of the various methods and compounds of the invention.

### Example 1

### Methods used to design probes of subsequent examples.

The candidate Gram-negative probes RW04, DL04, DL05, and RDR278, the Gram-positive probe RW03, and the Bacteroides probe RDR279 were designed from data in the Genbank or EMBL nucleotide sequence libraries (Dams, et al., "Compilation of small ribosomal subunit RNA sequences", (1988) Nucleic Acids Research, Vol. 16, Supplement and Neefs, et al, "Compilation of small ribosomal subunit RNA sequences", (1990) 18, 2237-2317, Supplement and in a paper by C. Woese (C. R. Woese, "Bacterial evolution", (1987) Microbiological Reviews, 51 (2), 221-271).

The location of the probes was chosen based on the finding of regions in the 16S rRNA gene which contain "sequence signatures" unique to the various groups of bacteria (Woese reference). All six of these probes are located in the same region of the gene. The nucleotide sequence of the probes was designed based on the sequences available for each group of bacteria to be detected and compared to corresponding sequences in groups of bacteria that were to be excluded. For example, the Gram-positive probe was designed to match most of the sequences found in most Gram-positive bacteria and to differ from the corresponding sequences in Gram-negative bacteria.

The candidate universal bacterial probes RDR244 and RDR245 correspond to a highly conserved region of the 16S rRNA gene. Most of the probe sequence in this region is present in most of the bacterial species for which sequence information is available and is not present in the nuclear or mitochondrial DNA of eukaryotic species.

In addition, each of the oligonucleotides described above was examined for self complementarity (ability to form base pairs with itself) using a computer program called FOLD in the University of Wisconsin series of programs. The position of the oligonucleotide probe was chosen to minimize the formation of secondary, structure where it was possible to do so while still maintaining the desired specificity.

The E. coli/enteric bacteria probe was designed from data in Genbank. The probe was designed using the following steps:

First, the nucleotide sequence from bp 1430 to 1536 (as specified in the Neefs reference) (within the 370 bp region bounded by amplification primers RW01 and DG74) for E. coli and Proteus vulgaris was compared to that of a panel of nonenteric species, including Neisseria gonorrhoeae, Pseudomonas aeruginosa, and Pseudomonas testosteroni. Regions where differences in the sequence occurred were noted and used to design a candidate probe.

Second, the candidate probe was compared with the corresponding nucleotide sequence of more phylogenetically diverse species listed in Genbank or EMBL to ensure that the candidate oligonucleotide would not detect other species.

Third, the oligonucleotide was examined for self complementarity (ability to form base pairs with itself) using a computer program called OLIGO, (National Biosciences, Hamel, NN). The position of the oligonucleotide probe was chosen to minimize the formation of secondary structure where it was possible to do so while still maintaining the desired specificity.

### Example 2

### Detection of Gram-negative bacteria Using PCR and Probe DL04

PCR amplification of gram-negative bacterial DNA was accomplished as follows. The primers utilized are as shown in Figure 1.

A standard PCR 2x mix was made containing the following for amplifying a target sequence for both gram-positive and gram-negative bacteria :

| | |
|---|---|
| 10x standard PCR buffer | 10.0 ml |
| 50 mM MgCl₂ | 1.0 ml |
| dNTP's (2.5 mM total dNTP's) | 2.5 ml |
| primer RW01 (50 mM) | 1.0 ml |
| primer DG74 (50 mM) | 1.0 ml |
| H₂O | 35.0 ml |
| Taq DNA polymerase (5 U/ml) | 0.5 ml |

The 10x standard PCR buffer contains:
100 mM Tris-HCl, pH8.3
500 mM KCl
15 mM MgCl

A. 50 ml of a gram-negative bacterial DNA sample was mixed together with 50 ml of the PCR 2x mix.

The reaction mixture was placed in a 0.5 ml microfuge tube and the tube was placed in a thermal cycler manufactured by Perkin-Elmer. A two-step PCR cycle was used and the thermocycler was set as follows:
1. Time delay file - 5 minutes at 95°C
2. Thermocycle file - 95°C for 25 seconds 55°C for 25 seconds, each incubation for 25 to 35 cycles.
3. Time delay file - 10 minutes at 72°C.

### B. Detection of amplified products

After the amplification reaction is complete, 5 ml of the 100 ml PCR reaction was mixed with 1 ml of 10x DNA dye buffer (50% sucrose, 10mM Tris, pH 7.5, 1 mM EDTA, 1.0% SDS, 0.05% bromphenol blue). The sample was loaded onto a 2% Nusieve agarose, 0.5% Seakem agarose, 1x TBE (45 mM Tris-borate, 1mM EDTA) gel. After running the bromphenol blue dye front to the bottom of the gel, the gel was stained with ethidium bromide (5 mg/ml), washed in water and photographed under UV light using a Polaroid camera and an orange filter.

The size of the PCR product is approximately 370 bp.

### C. Transfer of amplified DNA to nylon membrane

After photography of the gel, the gel was soaked in 0.25 N HCl for 10 minutes at room temperature. The gel was rinsed in water and then soaked in solution of 0.5 N NaOH, 1.5 M NaCl for 30 minutes. The gel was then rinsed in water and then soaked in a solution of 1 M Tris, pH 7.5, 1.5 M NaCl for 30 minutes.

DNA was then transferred to a nylon membrane (Pall Biodyne) presoaked in water by one of two ways: (1) vacuum transfer using a Stratagene Stratavac vacuum blotter or (2) capillary transfer by the method of Southern.

After transfer, DNA was fixed to the membrane using UV light in a Stratagene Stratalinker.

### D. Radioactive labeling of oligonucleotide probe DL04 (Figure 3).

Oligonucleotide DL04 was labeled using T₄ polynucleotide kinase in the following reaction mix:

| | |
|---|---|
| γ-32-P ATP | 10 ml |
| 10x kinase buffer | 2.5 ml |
| oligonucleotide (10mM) | 2.0 ml |
| H₂O | 8.5 ml |
| T4 polynucleotide kinase | 2.0 ml |

10x kinase buffer contains:
500 mM Tris, pH 8
100 mM MgCl₂
50 mM DTT

The kinase reaction mixture was incubated for 30 minutes at 37°C. 5.6 of 0.25 M EDTA and 169.4 ml of H₂O were added to stop the reaction. This mixture was loaded onto a 1.0 ml capacity column of Biogel P4 and spun in a tabletop centrifuge for 5 minutes at 5,000 rpm to separate the labeled oligonucleotide from the unincorporated radioactivity. 1ml of the eluate from the column was counted in a scintillation counter without added scintillation fluid (Cerenkov counting) to obtain an estimate of the level of incorporation of radioactivity. A volume giving approximately 1 x 10⁶ cpm was used for each blot in the subsequent hybridization.

### E. Hybridization of probes with DNA

The DNA blots were prehybridized in a mixture of 5x SSPE, 0.5% SDS at 60°C (1X SSPE = 0.18 M NaCl, 10 mM NaPO₄, pH 7.4, 1 mM EDTA). The labeled oligonucleotide probe was added to 7.5 ml of 5x SSPE, 0.5% SDS and mixed. The solution was added to the plastic bag containing the presoaked blot. The blot was incubated for 1 to 18 hours at 60°C.

The blot was removed from the plastic bag and placed in a solution of 2x SSPE, 0.1% SDS and washed for 10 minutes at room temperature. The blot was then washed in a solution of 3 M tetramethylammonium chloride (TMACl), 50 mM Tris, pH8 and 0.2% SDS for 10 minutes at 64°C for gram-negative probe DL04.

The blot was air-dried and wrapped in Saran wrap and placed in a X-ray film holder with a sheet of Kodak XAR-5 X-ray film with or without an intensifying screen for 1 to 72 hours at -70°C.

### Example 3

### Detection of Gram-negative bacteria - comparison of probes RW04 and DL04

Candidate Gram-negative probe RW04 was labeled with ³²P and hybridized to PCR products from various bacterial DNA's as described for Gram-negative probe DL04 in Example 2, except that the wash in TMACl was done at 62°C. The Southern blot results of the hybridizations are summarized in Table 1. The data show that the hybridization results obtained by the two probes are different even though both probes were designed to be Gram-negative "universal" probes. RW04 gave a positive signal for many Gram-positive species it should not have detected; while DL04 gave positive signals for only the Gram-negative species it should have detected (with the exception of T. maritima and T. thermophilus, which are not human pathogens). DL04 was therefore selected as a probe useful for detecting Gram-negative bacteria. Further testing (Table 2) indicated that DL04 did not detect all gram-negative species. A second candidate Gramnegative probe, RDR278, was tested as follows in Example 4.

### Example 4

### Detection of Gram-negative bacteria with probe RDR278

Gram-negative bacteria were detected using the same methods and materials as Example 2 including the wash in TMACl which was done at 64°C.

Gram-negative probe RDR278 was labeled with ³²P and hybridized to PCR products from various bacterial DNA's. The data are presented in Table 2. RDR278 gave a positive hybridization signal for most of the species not detected by DL04. The exception among the species tested was Bacteroides fragilis, for which a separate probe was designed. Therefore, it is observed that the combination of Gram-negative probes DL04 and RDR278 detect the majority of Gram-negative bacteria tested.

### Example 5

### Detection of Bacteroides fragilis with probe RDR279

The methods and materials of Example 2 were used including the wash in TMACl, which was done at 64°C.

Probe RDR279, corresponding to a region which is a sequence signature for Bacteroides (ref.), was labeled with 32p and hybridized to PCR products from various bacterial DNA's. Table 2 summarizes the results of testing of RDR279 against other bacterial species. The probe detected Bacteroides fragilis and did not give a reaction with any of the other bacterial species tested.

## Claims

1. A probe for detection or identification of pathogenic Gram-negative bacteria causing septicemia, containing a nucleotide sequence selected from the group consisting of DL04: 5'-GACGTAAGGGCCATGATGACTTGACGTC-3', RDR278: 5'-GACGTAAGGGCCATGAGGACTTGACGTC-3', RDR279: 5'-GACGTAAGGGCCGTGCTGATTTGACGTC-3', or a nucleotide sequence complementary thereto, which probe hybridizes under stringent conditions to a nucleotide sequence unique to Gram-negative bacteria.

2. A probe according to claim 1 containing a nucleotide sequence selected from the group consisting of DL04: 5'-GACGTAAGGGCCATGATGACTTGACGTC-3', RDR278: 5'-GACGTAAGGGCCATGAGGACTTGACGTC-3', or a sequence complementary thereto.

3. A probe according to claim 2 consisting of a nucleotide sequence selected from the group consisting of DL04: 5'-GACGTAAGGGCCATGATGACTTGACGTC-3', RDR278:    5'-GACGTAAGGGCCATGAGGACTTGACGTC-3', or a sequence complementary thereto.

4. A probe according to claim 1 for detection or identification of Bacteroides bacteria, containing nucleotide sequence RDR279: 5'-GACGTAAGGGCCGTGCTGATTTGACGTC-3' or the sequence complementary thereto.

5. A probe according to claim 4 consisting of nucleotide sequence RDR279: 5'-GACGTAAGGGCCGTGCTGATTTGACGTC-3' or the sequence complementary thereto.

6. A PCR kit for the detection or identification of pathogenic Gram-negative bacteria comprising a first container and a second container wherein the first container contains primers capable of amplifying a target region of a polynucleotide sequence within bacteria, and the second container contains one or more probes according to any one of claims 1 to 5.

7. A method for determining the presence of a bacterial polynucleotide from a pathogenic Gram-negative bacterium in a sample suspected of containing said bacterial polynucleotide, wherein said bacterial polynucleotide comprises a selected target region, said method comprising :
(a) amplifying the target region, if any, to a detectable level;
(b) incubating the amplified target region, if any, with a probe according to any of claims 1 to 5 under conditions which allow specificity of hybrid duplexes; and
(c) detecting hybrids formed between the amplified target region, if any, and the probe.

8. The method of claim 7 wherein the target region is amplified by means of the Polymerase Chain Reaction (PCR).

## Patentansprüche

1. Sonde zum Nachweis oder zur Identifizierung von pathogenen Gram-negativen Bakterien, die eine Septikemie verursachen, enthaltend eine Nucleotidsequenz ausgewählt aus der Gruppe bestehend aus: DL04: 5'-GACGTAAGGGCCATGATGACTTGACGTC-3' RDR278: 5'-GACGTAAGGGCCATGAGGACTTCAGCTG-3' RDR279: 5'-GACGTAAGGGCCGTGCTGATTTGACGTC-3' oder eine dazu komplementäre Nucleotidsequenz, wobei die Sonde unter stringenten Bedingungen mit einer Nucleotidsequenz hybridisiert, die einzigartig für Gram-negative Bakterien ist.

2. Sonde nach Anspruch 1, enthaltend eine Nucleotidsequenz, ausgewählt aus der Gruppe bestehend aus: DL04: 5'-GACGTAAGGGCCATGATGACTTGACGTC-3', RDR278: 5'-GACGTAAGGGCCATGAGGACTTGACGTC-3'. oder eine dazu komplementäre Sequenz.

3. Sonde nach Anspruch 2, bestehend aus einer Nucleotidsequenz, ausgewählt aus der Gruppe bestehend aus: DL04: 5'-GACGTAAGGGCCATGATGACTTGACGTC-3', RDR278: 5'-GACGTAAGGGCCATGAGGACTTGACGTC-3', oder einer dazu komplementären Sequenz.

4. Sonde nach Anspruch 1 zum Nachweis oder zur Identifizierung von Bacteroides-Bakterien, enthaltend die Nucleotidsequenz: RDR279: 5'-GACGTAAGGGCCGTGCTGATTTGACGTC-3' oder die dazu komplementäre Sequenz.

5. Sonde nach Anspruch 4, bestehend aus der Nucleotidsequenz: RDR279: 5'-GACGTAAGGGCCGTGCTGATTTGACGTC-3' oder der dazu komplementären Sequenz.

6. PCR-Kit zum Nachweis oder zur Identifizierung von pathogenen Gram-negativen Bakterien, umfassend einen ersten Behälter und einen zweiten Behälter, wobei der erste Behälter Primer enthält, die zur Amplifikation eines Zielbereiches einer Polynucleotidsequenz innerhalb der Bakterien fähig sind, und der zweite Behälter eine oder mehrere Sonden nach einem der Ansprüche 1 bis 5 enthält.

7. Verfahren zur Bestimmung der Gegenwart eines bakteriellen Polynucleotids von einem pathogenen Gram-negativen Bakterium in einer Probe, von der vermutet wird, dass sie das bakterielle Polynucleotid enthält, wobei das bakterielle Polynucleotid einen ausgewählten Zielbereich umfasst, wobei das Verfahren umfasst:
a) Amplifizieren des Zielbereiches, falls vorhanden, bis zu einer nachweisbaren Menge;
b) Inkubieren des amplifizierten Zielbereiches, falls vorhanden, mit einer Sonde nach einem der Ansprüche 1 bis 5 unter Bedingungen, die eine Spezifität der Hybriddoppelstränge erlauben; und
c) Nachweisen der Hybride, die zwischen dem amplifizierten Zielbereich, falls vorhanden, und der Sonde gebildet wurden.

8. Verfahren nach Anspruch 7, wobei der Zielbereich mit Hilfe der Polymerase-Ketten-Reaktion (PCR) amplifiziert wird.

## Revendications

1. Sonde pour la détection ou l'identification de bactéries Gram-négatives pathogènes provoquant la septicémie, contenant une séquence nucléotidique choisie dans l'ensemble comprenant : DL04: 5'-GACGTAAGGGCCATGATGACTTGACGTC-3', RDR278: 5'-GACGTAAGGGCCATGAGGACTTGACGTC-3', RDR279: 5'-GACGTAAGGGCCGTGCTGATTTGACGTC-3', ou un séquence nucléotidique qui lui est complémentaire, laquelle sonde s'hybride dans des conditions stringentes à une séquence nucléotidique propre aux bactéries Gram-négatives.

2. Sonde selon la revendication 1, contenant une séquence nucléotidique choisie dans l'ensemble comprenant DL04: 5'-GACGTAAGGGCCATGATGACTTGACGTC-3', RDR278: 5'-GACGTAAGGGCCATGAGGACTTGACGTC-3', ou une séquence qui lui est complémentaire.

3. Sonde selon la revendication 2, consistant en une séquence nucléotidique choisie dans l'ensemble comprenant DL04: 5'-GACGTAAGGGCCATGATGACTTGACGTC-3', RDR278: 5'-GACGTAAGGGCCATGAGGACTTGACGTC-3', ou une séquence qui lui est complémentaire.

4. Sonde selon la revendication 1, pour la détection ou l'identification de bactéries de type Bacteroides, contenant la séquence nucléotidique RDR279: 5'-GACGTAAGGGCCGTGCTGATTTGACGTC-3' ou la séquence qui lui est complémentaire.

5. Sonde selon la revendication 4, consistant en la séquence nucléotidique RDR279: 5'-GACGTAAGGGCCGTGCTGATTTGACGTC-3' ou la séquence qui lui est complémentaire.

6. Kit pour PCR, pour détecter ou identifier des bactéries Gram-négatives pathogènes, comprenant un premier récipient et un deuxième récipient, où le premier récipient contient des amorces capables d'amplifier une région cible d'une séquence polynucléotidique se trouvant à l'intérieur d'une bactérie, et le deuxième récipient contient une ou plusieurs sondes selon l'une ou plusieurs des revendications 1 à 5.

7. Procédé pour déterminer la présence d'un polynucléotide bactérien provenant d'une bactérie Gram-négative pathogène dans un échantillon que l'on suspecte de contenir ledit polynucléotide bactérien, où ledit polynucléotide bactérien comprend une région cible sélectionnée, ledit procédé comprenant :
(a) l'amplification, jusqu'à un niveau détectable, de l'éventuelle région cible ;
(b) l'incubation de l'éventuelle région cible amplifiée, à l'aide d'une sonde selon l'une quelconque des revendications 1 à 5, dans des conditions qui permettent la spécificité de duplex hybrides ; et
(c) la détection d'hybrides formés entre l'éventuelle région cible amplifiée et la sonde.

8. Procédé selon la revendication 7, dans lequel la région cible est amplifiée au moyen d'une réaction en chaîne par polymérase (PCR).
